# EUROPÄISCHE PATENTSCHRIFT

(11) **EP 2 266 548 B1**
(45) Veröffentlichungstag und Bekanntmachung des Hinweises auf die Patenterteilung: **20.05.2020**
(21) Anmeldenummer: 10180656.0
(22) Anmeldetag: 10.11.1999
(51) Int. Cl.: A61K 9/72, A61K 47/12

(54) **TROCKENPULVER ZUR INHALATION**
DRY POWDER FOR INHALATION
POUDRE SÈCHE POUR INHALATION

(30) Priorität: 13.11.1998 CH 228698
(43) Veröffentlichungstag der Anmeldung: 29.12.2010
(62) Teilanmeldung aus: 07116946.0
(73) Patentinhaber: Jagotec AG, 4132 Muttenz (CH)
(72) Erfinder: Keller, Manfred, 81379 München (DE); Müller-Walz, Rudi, 79650, Schopfheim (DE)
(74) Vertreter: Haile, Alison Victoria

(56) Entgegenhaltungen:
- EP-A1- 0 239 798
- EP-A2- 0 272 772
- WO-A1-96/23485
- PEART J ET AL: "MULTICOMPONENT PARTICLE INTERACTIONS IN DRY POWDER AEROSOLS", PHARMACEUTICAL RESEARCH, KLUWER ACADEMIC PUBLISHERS, NEW YORK, NY, US, Bd. 14, Nr. 11-S, 1. Januar 1997 (1997-01-01), XP001030455, ISSN: 0724-8741
- Nuha Mohammed Kassem: "Generation of deeply inspirable clouds from dry powder mixtures", 1990, XP002687372, Seiten 187-191, * Seite 188, Zeile 10 - Zeile 14 * * Seite 190, Zeile 4 - Zeile 9 * * Seite 190, Zeile 15 - Zeile 25 *
- H.V VAN KAMP; G.K. BOLHUIS; A.H DE BOER; C.F. LERK; L. LIE-A-HUEN: "The role of water uptake of tablet disintegration", PHARMACEUTICA ACTA HELVETIAE, Bd. 61, Nr. 1, 1986, Seiten 22-29, XP002687373, ISSN: 0031-6865
- MEAKING B J ET AL: "The effect of flow rate on drug delivery from Pulvinal, a high-resistance dry powder inhaler", JOURNAL OF AEROSOL MEDICINE, MARY ANN LIEBERT, INC., NEW YORK, US, Bd. 11, Nr. 3, 1. Januar 1998 (1998-01-01) , Seiten 143-152, XP008119278, ISSN: 0894-2684, DOI: 10.1089/JAM.1998.11.143 [gefunden am 2009-01-30]

## Beschreibung

Die Erfindung betrifft die Verbesserung der Feuchtigkeitsbeständigkeit von Trockenpulver-Formulierungen zur Inhalation in Multidosis-Trockenpulverinhalatoren wie in Anspruch 1 definiert.

Trockenpulver-Formulierungen zum Inhalieren müssen eine Reihe von einander teilweise widersprechenden Anforderungen erfüllen, wobei insbesondere die folgenden zu beachten sind:
Der Wirkstoff muss inhalierbar sein. Um in die Lunge gelangen zu können, muss er in Partikeln von ca. 1 bis 10 µm Grösse vorliegen. Solche mikrofeinen Partikel können beispielsweise durch Mikronisierung, kontrollierte Ausfällung aus geeigneten Solventien oder durch Sprühtrocknung erzielt werden, wenn die Verfahrensbedingungen geeignet gewählt, kontrolliert und ausgeführt werden. Mikrofeine Partikel haben jedoch ein sehr ungünstiges, d.h. grosses Verhältnis von Oberfläche zu Volumen bzw. Masse und daher eine grosse Oberflächenenergie. Dies äussert sich in starken Adhäsions- und Kohäsionstendenzen, die wiederum zu schlechten Fliesseigenschaften und zu Pulveraggregation führen. Derartige mikrofeine Pulver sind deshalb schwer zu handhaben und werden stark beeinflusst durch elektrostatische Aufladung, Bearbeitung, Luftfeuchtigkeit und dergleichen.

Um eine konsistente Herstellung der Formulierung, eine maschinelle Befüllung des Pulverinhalators und korrektes Dosieren und Freisetzen durch den Pulverinhalator zu gewährleisten, muss das Pulver frei fliessend sein. Gute Fliesseigenschaften erwartet man in der Regel bei genügend grossen, möglichst kugelförmigen Partikeln, die eine geringe Oberflächenenergie und kleine Kontaktflächen haben.

Bei Pulverinhalatoren mit einem Reservoir wird die fertige Arzneizubereitung in Form eines Pulverbetts in das Vorratsgefäss gefüllt. Eine Dosis wird durch eine geeignet ausgebildete Dosiervorrichtung entnommen. Die Entnahme erfolgt volumetrisch. Das genaue volumetrische Dosieren der Zubereitung erfordert für die meisten Wirkstoffe eine Verdünnung derselben mit einem pharmazeutisch inaktiven Hilfsstoff, um eine den Anforderungen an die Dosiergenauigkeit genügende, dosierbare Einheitsmenge zu erhalten.

Für Pulverinhalatoren, die das Medikament aus vordosierten Einheiten, z.B. Kapseln oder Blistern, freisetzen, gilt die gleiche Einschränkung für den reibungslosen Betrieb der Befüllungsmaschinen dieser Einheitsdosen.

Bei einem Multidosis-Trockenpulverinhalator, der ein Pulverreservoir enthält, aus dem die einzelnen Dosen über einen Dosiermechanismus entnommen werden, ist das pulverförmige Medikament in der Regel in Kontakt mit der Umgebungsluft und kann so durch Luftfeuchtigkeit beeinflusst werden. Die Qualität des Medikaments und des Inhalationssystems darf sich jedoch durch den Einfluss von äusseren Faktoren während der vorgesehenen Lagerzeit und bis zum Aufbrauchen der Packung nicht wesentlich verschlechtern.

Um diesen Anforderungen zu genügen, werden die inhalierbaren, d.h. in mikrofeinen Partikeln vorliegenden Bestandteile (Wirkstoffe) mit pharmakologisch inaktiven Substanzen gemischt, um fliessfähige Pulver zu erhalten. Die Verdünnung wird dabei so gewählt, dass die vom Pulverinhalator ausgebrachte Menge genau die gewünschte Dosis enthält. Der überwiegende Anteil des pharmakologisch inaktiven Hilfsstoffes liegt dabei absichtlich in einer Partikelgrösse vor, die nicht inhalierbar ist. Er dient nicht nur zum Verdünnen, sondern auch zum Einstellen einer akzeptablen, möglichst einer guten bis sehr guten Fliessfähigkeit der Pulvermischung. Er ist im Fall dieser sogenannten interaktiven oder geordneten Mischungen die Trägersubstanz, an die die mikrofeinen Wirkstoffpartikel durch Adhäsion gebunden werden, um so eine geeignete Mischgüte, d.h. Homogenität der Mischung, zu erreichen und aufrecht zu erhalten. Durch das Mischverfahren kann sich die Partikelgrösse des Trägers auch so ändern, dass ein bestimmter Anteil inhalierbar wird. Die Partikelgrösse des eingesetzten Trägers richtet sich dabei in der Regel nach den Anforderungen und Gegebenheiten des Pulverinhalators, der für die Applikation der Formulierung vorgesehen ist. Für diese Mischungen gilt, dass während allen notwendigen Verarbeitungs-, Transport-, Lager- und Dosiervorgängen keine Entmischung stattfinden darf, d.h. die Wirkstoffpartikel sich nicht von ihren Trägerteilchen ablösen dürfen. Während dem Dispergieren im Inhalator, ausgelöst durch den Atemfluss des Patienten, müssen die Wirkstoffpartikel aber möglichst effektiv, d.h. möglichst quantitativ, abgelöst werden, um inhaliert werden zu können. Der Träger (Carrier) ist in den meisten Fällen Lactose, kann aber auch Mannitol, Trehalose oder ein anderes geeignetes Trägermaterial sein. In manchen auf dem Markt erhältlichen Inhalatoren ist auch Glucose als Trägermaterial enthalten.

Es ist bekannt, dass die Fliesseigenschaften geordneter Mischungen in der Hauptsache von den physikalisch-chemischen Eigenschaften des Trägers, der ja in der Regel im Überschuss beigemischt wird, abhängen. Ebenso ist bekannt, dass die Effektivität der Freisetzung der inhalierbaren Primärpartikel des Wirkstoffs durch Scherkraft neben den physikalisch-chemischen, stoffspezifischen Eigenschaften des Wirkstoffes und den physikalischen, insbesondere aerodynamischen Eigenschaften des Pulverinhalators vor allem auch von den Eigenschaften des Trägers abhängt. Als analytische Messgrösse wird dazu in vitro in sogenannten Kaskadenimpaktoren oder Liquid Impingern, wie sie in verschiedenen Pharmakopöen beschrieben sind, die Menge Wirkstoff in feinen, inhalierbaren Partikeln (Feinpartikeldosis bzw. Fine Particle Dose, nachfolgend auch mit FPD bezeichnet) bzw. der Feinpartikelanteil (Fine Particle Fraction, nachfolgend auch mit FPF bezeichnet) bezogen auf die Gesamtmenge an abgegebenem Wirkstoff bestimmt.

Kürzliche Arbeiten zeigen, dass die FPF umso höher ist, je kleiner die Partikelgrösse der zugemischten Lactose ist [M.J. Clarke, U.J. Potter, P. Lucas, M.J. Tobyn und J.N. Staniforth: Posterpräsentation auf der Konferenz "Drug Delivery to the Lungs VIII" der Aerosol Society, London, 15.-16.12.1997; und P. Lucas, M.J. Clarke, K. Anderson, M.J. Tobyn und J.N. Staniforth (1998): Vortrag auf der Konferenz "Respiratory Drug Delivery VI", Hilton Head Island, 3.-7.5.1998, veröffentlicht in: R.N. Dalby, P.R. Byron und S.J. Farr (Herausgeber): Respiratory Drug Delivery VI, Interpharm Press, 1998, 243 ff.]. Dieses Verfahren stösst jedoch an eine natürliche Grenze, da die Fliessfähigkeit mit kleineren Partikeln rasch unzureichend wird.

Es konnte ebenfalls gezeigt werden, dass beim Vergleich gleicher Siebfraktionen von verschiedenen Lactosequalitäten eine umkristallisierte Lactose die höhere FPF erzielte [N.M. Kassem und D. Ganderton: J. Pharm. Pharmacol. 42 (1990), 11 ff. (Suppl.) und EP-B-0 464 171]. Dieser Effekt beruht auf der Tatsache, dass die Wirkstoffpartikel bevorzugt an Fehlstellen, Risse und Brüche, d.h. an besonders aktivierte Zentren (sogenannte "active sites" oder "hot spots") der Trägerpartikel anhaften. Die Adhäsionskräfte sind an diesen aktivierten Zentren am grössten und damit auch die Ablösung während der Inhalation am wenigsten wahrscheinlich. Es konnte nun durch elektronenmikroskopische Aufnahmen gezeigt werden, dass die umkristallsierte Lactose sehr viel regelmässiger ist als die handelsübliche Ware.

Weiterhin ist bekannt, dass auch kristallines α-Lactose-Monohydrat einen geringen Anteil amorphe Lactose enthält, die die regelmässige Kristallstruktur stört und damit für aktivierte Stellen auf der Kristalloberfläche sorgt [G. Buckton und P. Darcy: Int. J. Pharm. 123 (1995), 265 ff.; E.M. Phillips: Int. J. Pharm. 149 (1997), 267 ff.]. Wasser kann bei erhöhter Luftfeuchtigkeit bevorzugt an diese amorphen Zentren anlagern und als Weichmacher eine Umwandlung in die thermodynamisch stabilere Kristallform verursachen [B.C. Hancock und G. Zografi: J. Pharm. Sci. 86 (1997), 1 ff.]. Das wiederum hat zur Folge, dass die Lagerstabilität derartiger Pulverzubereitungen bei erhöhter Luftfeuchte begrenzt ist.

In WO-A-95/11666 wurde vorgeschlagen, die aktiven Zentren durch Zusatz von mikrofeiner Lactose abzusättigen mit dem Ziel, dem Wirkstoff beim Herstellen der endgültigen Mischung nur noch weniger energiereiche Bindungsstellen auf der Lactose zur Verfügung zu stellen. Da die Ablösung während der Inhalation demzufolge weniger Energie benötigt, sollte die FPF signifikant steigen, was eindeutig nachgewiesen werden konnte. Gleiches gilt auch für das Verfahren, das in WO-A-93/11746 beschrieben ist.

In J. Pharm. Pharmacol. 34: 141-145 (1982) wurde ferner gefunden, dass der Zusatz einer dritten Pulverkomponente zu einer vorher gebildeten geordneten Mischung aus Salicylsäure (1%) und Sucrose infolge Ladungswechselwirkungen die physikalische Stabiltät von ternären Mischungen in unterschiedlicher Weise beeinflussen kann. Die Zugabe von 0,5-4,0% Magnesiumstearat beeinträchtigte die Adhäsion der Salicylsäure-Partikel an den Sucrose-Träger, wobei sich der Anteil schwach gebundener Wirkstoffpartikel mit zunehmender Magnesiumstearat-Konzentration erhöhte. Dieser Befund wurde einer Änderung der Ladungswechselwirkungen auf der Oberfläche der Sucrose-Trägerpartikel als Folge der positiven elektrostatischen Ladung des Magnesiumstearats und der negativen Ladung der Salicylsäure- und Sucrose-Partikel zugeschrieben. Auf diesen Effekt und die Tatsache, dass die Zugabe einer dritten Komponente, die bevorzugt an den Trägerpartikeln anlagert, die Wirkstoffpartikel von ihren Adhäsionsstellen verdrängen kann, wurde bereits in J. Pharm. Pharmacol. 31: 800 (1979) hingewiesen. Demgegenüber wurde durch Zugabe von 2% Maisstärke die Adhäsion der Wirkstoffpartikel verstärkt und die an Sucrose haftende Wirkstoffmenge erhöht, während durch Zugabe von 2% Talk die Adhäsionskräfte zwischen den Teilchen allgemein erhöht wurden. Ähnliche Effekte wurden auch von N.M. Kassem [Doktorarbeit DX187842, Universität London, 1990] gefunden und ebenfalls durch die elektrostatischen Eigenschaften der Bestandteile erklärt. In dieser Doktorarbeit werden Multidosis-Trockenpulverinhalatoren nicht gennant und sie befasst sich nicht mit der Feuchtigkeitsanfälligkeit solcher Formulierungen.

In WO-A-87/05213 wurde andererseits vorgeschlagen, zur Herstellung von Inhalationspulvern Träger, bestehend aus Mikrokörnchen eines Konglomerats eines oder mehrerer fester wasserlöslicher Verdünnungsmittel, wie Lactose, Xylitol, Mannitol, Arabinose oder Dextran, mit einem Schmiermittel, wie Magnesiumstearat, Natriumbenzoat, kolloidalem Silika, hydriertem Öl oder Fettsubstanzen, zu verwenden. Die Mikrokörnchen haben vorzugsweise eine Korngrösse von 30-150 µm und werden dadurch hergestellt, dass das Schmiermittel einer wässrigen Lösung eines Teils des festen Verdünnungsmittels zugesetzt, das restliche Verdünnungsmittel zusammen mit dieser Mischung granuliert und das erhaltene Granulat gesiebt wird. Die Verwendung solcher Träger soll u.a. verbesserte Fliesseigenschaften und verbesserte selbstschmierende Eigenschaften ermöglichen.

Die EP-A-0272772 beschreibt weiterhin pharmazeutische Zusammensetzungen in Pulverform zur Inhalation mit den mikronisierten Wirkstoffen Beclomethason-dipropionat bzw. Salbutamol.

Die WO 9623485 offenbart Pulverformulierungen zur Verwendung in Trockenpulver-Inhalatoren, welche neben Wirkstoffpartikeln Trägerpartikel mit Zusatz von Beispielsweise Magnesiumstearat enthalten.

In Journal of Aerosol Medicine 11(3), pp. 143-152 (1998) wird der Trockenpulverinhalator "Pulvinal" und eine darin enthaltene Trockenpulverformulierung mit Beclomethasondipropionat und 0.25 Gew-% Magnesiumstearat untersucht.

Es hat sich jedoch gezeigt, dass Pulvermischungen, insbesondere interaktive Pulvermischungen, sensititv gegenüber der Feuchtigkeit in der Umgebungsluft sind. Sie sind daher für den Einsatz in einem Multidosis-Trockenpulverinhalator, der ein Pulverreservoir enthält, nur bedingt geeignet, da dieser normalerweise keine dichte Verpackung im Sinne einer hermetischen Absperrung von Wasserdampf darstellt. Dies äussert sich meist in einem dramatischen Abfall des inhalierbaren Anteils der abgegebenen Dosis, die in vitro als FPD oder FPF bestimmt wird. Der Abfall beruht auf einer stärkeren Adhäsion der mikroniserten Wirkstoffpartikel an den Trägerpartikeln, da ab einer relativen Luftfeuchtigkeit von ca. 60 % durch Wasserdampfkondensation sogenannte Flüssigkeitsbrücken in den Zwischenräumen entstehen, die zu einer stärkeren Bindungsenergie beitragen. Visuell sichtbares Zeichen für diesen Vorgang sind Krusten- oder Klumpenbildung, die aber nicht in jedem Fall notwendigerweise beobachtet werden müssen. Der Vorgang ist irreversibel, da beim Auftrocknen der Flüssigkeitsbrücken sogenannte feste Brücken entstehen. Unter anderem ist die Wasseraufnahmetendenz bzw. das Wassersorptionsvermögen der beteiligten Stoffe mitentscheidend für das Ausmass der Verschlechterung der Pulvereigenschaften bei hoher Luftfeuchtelagerung.

Der Erfindung betrifft die Verwendung von staubförmigem Magnesiumstearat wie in Anspruch 1 definiert. Spezielle AUsführungsformen der Erfindung sing Gegenstand der Patentsansprüche 2 bis 10.

Es hat sich nämlich überraschenderweise gezeigt, dass Magnesiumstearat in der Lage ist, den Einfluss von eindringender Feuchtigkeit auf die FPD und die FPF während der Lagerung des Inhalationspulvers zu minimieren, d.h. eine durch Feuchtigkeit bedingte Verschlechterung der FPD und der FPF zu verhindern oder zumindest erheblich zu verlangsamen, und die Trockenpulver-Formulierung zu stabilisieren. Die ursprüngliche Qualität der Arzneizubereitung bleibt also auch bei Lagerung unter extremen Bedingungen der Temperatur und Feuchte wesentlich besser als bei herkömmlichen Präparaten. Die Verbesserung äussert sich meist auch dadurch, dass der Einfluss von Feuchtigkeit auf das Massenmittel des aerodynamischen Teilchendurchmessers (Mass Median Aerodynamic Diameter, nachfolgend auch als MMAD bezeichnet) und auf die Genauigkeit und Reproduzierbarkeit der abgegebenen Dosis verhindert oder stark verlangsamt werden kann. Diese Effekte sind speziell für feuchtigkeitsempfindliche Wirkstoffe besonders ausgeprägt, da eine eventuelle Hygroskopizität des Wirkstoffes die Wasseraufnahme und damit die Ausbildung der Flüssigkeitsbrücken begünstigt. Darüber hinaus führt die Verwendung von Magnesiumstearat in der Regel zu einer generellen Verbesserung der FPD und der FPF. Es ist denkbar, dass das Magnesiumstearat neben einem generellen Feuchtigkeitsschutz die Trägermaterialien und Wirkstoffe auch in der Weise stabilisiert, dass unerwünschte morphologische Phasenübergänge unterbunden oder verlangsamt werden.

Die Verwendung des Magnesiumstearats ermöglicht die Verbesserung der Feuchtigkeitsbeständigkeit, d.h. zur Verringerung der Sensitivität gegenüber Luftfeuchtigkeit, von Trockenpulver-Formulierungen zur Inhalation. Die Verwendung von Magnesiumstearat bewirkt dementsprechend eine Verbesserung der Lagerstabilität und insbesondere eine Verringerung des Einflusses von eindringender Feuchtigkeit auf die FPF (und die FPD), was die Aufrechterhaltung einer hohen FPD und FPF auch unter vergleichsweise extremen Temperatur- und Feuchtigkeitsbedingungen gestattet.

Die erfindungsgemäss erhältlichen Trockenpulverformulierungen umfassen somit einen pharmazeutisch nicht wirksamen Träger in nicht inhalierbarer Teilchengrösse, einen fein verteilten pharmazeutischen Wirkstoff in inhalierbarer Teilchengrösse (d.h. mit einem mittleren Teilchendurchmesser von vorzugsweise höchstens 10 µm, insbesondere höchstens 5 µm) und - zur Verbesserung der Feuchtigkeitsbeständigkeit - Magnesiumstearat, und sie liegen vorzugsweise in Form von sogenannten interaktiven (oder geordneten oder adhäsiven) Mischungen vor. Gewünschtenfalls können die Trockenpulver-Formulierungen auch einen Anteil an Trägermaterial in inhalierbarer Teilchengrösse enthalten.

Der Ausdruck "interaktive Mischung" oder "geordnete Mischung" oder "adhäsive Mischung" ist dem Fachmann geläufig und umfasst im Rahmen der vorliegenden Erfindung Trockenpulver-Formulierungen, in denen der pharmakologisch inaktive Träger in einer Partikelgrösse vorliegt, die nicht inhalierbar oder überwiegend nicht inhalierbar ist, und in denen mikrofeine Wirkstoffpartikel durch Adhäsion an die Trägerpartikel gebunden (d.h. nicht im Träger z.B. in Form eines Granulates enthalten) sind.

Es wurde gefunden, dass sich Magnesiumstearat zur Verbesserung der Feuchtigkeitsbeständigkeit grundsätzlich beliebiger Trockenpulver-Formulierungen, unabhängig von der Art der Wirkstoffe und Trägermaterialien, eignet. Besonders ausgeprägt ist die Verbesserung jedoch im Falle von Trockenpulvern, deren Kombination von Wirkstoff und Träger - d.h. ohne Zusatz von Magnesiumstearat - eine hohe Sensitivität gegenüber dem Einfluss von Luftfeuchtigkeit aufweist und beispielsweise bei offener Lagerung bei 40°C und 75% relativer Luftfeuchtigkeit innert 10 Tagen eine Abnahme der FPF um mindestens 50% zeigt. Eine hohe Empfindlichkeit der FPF oder FPD auf Luftfeuchtigkeit wird häufig dann beobachtet, wenn der Wirkstoff in Form eines Salzes oder Esters vorliegt und/oder vergleichsweise hygroskopisch oder hydrophil ist.

Hygroskopisch ist in diesem Sinne ein Wirkstoff dann, wenn er bei einem Wasserdampfdruck in der Trocknungsluft > 0, d.h. in Kontakt mit Luft mit einem Feuchtigkeitsgehalt > 0% relativer Feuchte, nie vollständig austrocknet, sondern immer eine bestimmte Menge absorptiv gebundenes Wasser enthält [H. Sucker, P. Fuchs und P. Speiser: Pharmazeutische Technologie, Georg Thieme Verlag, Stuttgart, New York, 2. Auflage 1991, Seite 85]. Besonders vorteilhaft ist die erfindungsgemässe Verwendung von Magensiumstearat, wenn der Wirkstoff vergleichsweise hygroskopisch ist und beispielsweise bei Lagerung in Trocknungsluft mit einer relativen Feuchte von 50% mindestens etwa 0,5 Gew.-% adorptiv gebundenes Wasser aufnimmt bzw. behält.

Hydrophil ist ein Wirkstoffpulver dann, wenn es von Wasser leicht benetzt werden kann, wobei im Rahmen der vorliegenden Erfindung unter hydrophilen Wirkstoffpulvern insbesondere solche zu verstehen sind, die beispielsweise einen Benetzungswinkel von weniger als 90° aufweisen [Martin, Swarbrick und Cammarata: Physikalische Pharmazie, Wissenschaftliche Verlagsgesellschaft mbH, Stuttgart, 3. Auflage 1987, Seite 534]. Besonders vorteilhaft ist die erfindungsgemässe Verwendung von Magnesiumstearat im Falle von Wirkstoffpulvern, die einen Benetzungswinkel von weniger als 70° aufweisen.

Die Verwendung von Magnesiumstearat zur Verbesserung der Feuchtigkeitsbeständigkeit von Trockenpulver-Formulierungen ist somit besonders bevorzugt im Falle von Trockenpulver-Formulierungen, die einen pharmazeutischen Wirkstoff enthalten, der in Form eines Salzes oder Esters vorliegt und/oder bei Lagerung in Trocknungsluft mit einer relativen Feuchte von 50% mindestens etwa 0,5 Gew.-% adorptiv gebundenes Wasser aufnimmt bzw. behält und/oder einen Benetzungswinkel von weniger als 90°, insbesondere weniger als 70°, aufweist.

Die Verwendung von Magnesiumstearat ist für den Einsatz in Multidosis-Trockenpulverinhalatoren vorteilhaft, die ein Pulverreservoir enthalten, aus dem die einzelnen Dosen über einen Dosiermechanismus entnommen werden.

Der in den Formulierungen vorhandene Wirkstoff kann grundsätzlich ein beliebiger, in Trockenpulvern inhalativ verabreichbarer pharmazeutischer Wirkstoff sein. Damit der Wirkstoff inhalierbar ist, d.h. in die Lunge gelangen kann, muss er in Partikeln mit einem mittleren Teilchendurchmesser (gemessen als MMAD) von höchstens etwa 10 µm, beispielsweise etwa 1 bis 10 µm und vorzugsweise etwa 1 bis 6 µm, vorliegen. Solche mikrofeinen Partikel können in bekannter oder an sich bekannter Weise, beispielsweise durch Mikronisierung, kontrollierte Ausfällung aus geeigneten Solventien (z.B. auch aus superkritischem Kohlendioxid) oder durch Sprühtrocknung, erhalten werden, wenn die Verfahrensbedingungen geeignet gewählt, kontrolliert und ausgeführt werden.

Die Formulierungen können als Wirkstoff vorzugsweise ein Betamimetikum, wie Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenalin oder Hexoprenalin, ein Anticholinergikum, wie Tiotropium, Ipratropium, Oxitropium oder Glycopyrronium, ein Corticosteroid, wie Butixocart, Rofleponid, Budesonid, Ciclosenid, Mometason, Fluticason, Beclomethason, Loteprednol oder Triamcinolon, einen Leukotrienantagonisten, wie Andolast, Iralukast, Pranlukast, Imitrodast, Seratrodast, Zileuton, Zafirlukast oder Montelukast, einen Phosphodiesterase-Inhibitor, wie Filaminast oder Piclamilast, einen PAF-Inhibitor, wie Apafant, Forapafant oder Israpafant, einen Kaliumkanalöffner, wie Amilorid oder Furosemid, ein Schmerzmittel, wie Morphin, Fentanyl, Pentazozin, Buprenorphin, Pethidin, Tilidin, Methadon oder Heroin, ein Potenzmittel, wie Sildenafil, Alprostadil oder Phentolamin, ein Peptid oder Protein, wie Insulin, Erythropoietin, Gonadotropin oder Vasopressin, oder ein pharmazeutisch annehmbares Derivat oder Salz dieser Verbindungen enthalten. Im Falle von chiralen Wirkstoffen kann dieser in Form eines optischen Isomers, eines Diastereoisomerengemisches oder Racemates vorliegen. Gewünschtenfalls können die erfindungsgemässen Formulierungen zwei oder mehrere pharmazeutische Wirkstoffe enthalten.

Da die Feuchtigkeitsempfindlichkeit vor allem im Falle von Wirkstoffen, die als Salz oder Ester vorliegen, häufig ein grosses Problem darstellt, ist der Einsatz von Magnesiumstearat insbesondere bei Trockenpulver-Formulierungen vorteilhaft, die mindestens einen pharmazeutischen Wirkstoff in Form eines pharmazeutisch annehmbaren Salzes, beispielsweise ein Chlorid, Bromid, Iodid, Nitrat, Carbonat, Sulfat, Methylsulfat, Phosphat, Acetat, Benzoat, Benzolsulfonat, Fumarat, Malonat, Tartrat, Succinat, Citrat, Lactat, Gluconat, Glutamat, Edetat, Mesylat, Pamoat, Pantothenat oder Hydroxynaphthoat, oder einen pharmazeutischen Wirkstoff in Form eines pharmazeutisch annehmbaren Esters, beispielsweise ein Acetat, Propionat, Phosphat, Succinat oder Etabonat, enthalten.

Besonders bevorzugt ist die Verwendung von Magnesiumstearat in Trockenpulver-Formulierungen, die ein Betamimetikum und/oder ein Anticholinergikum und/oder ein Corticosteroid enthalten, und insbesondere in Trockenpulver-Formulierungen, die ein Betamimetikum und/oder ein Anticholinergikum und/oder ein Corticosteroid in Form eines pharmazeutisch annehmbaren Salzes oder Esters enthalten, beispielsweise ein Betamimetikum in Form eines Salzes, wie z.B. Levalbuterolsulfat, Formoterolfumarat, Formoteroltartrat, Salbutamolsulfat oder Salmeterolxinafoat (Salmeterol-1-hydroxy-2-naphthoat), oder ein Anticholinergikum in Form eines Salzes, wie z.B. Oxitropiumbromid, Glycopyrrolat (Glycopyrroniumbromid), Ipratropiumbromid oder Tiotropiumbromid, oder ein Corticosteroid in Form eines Esters, wie z.B. Beclomethason-dipropiponat, Fluticason-propionat, Triamcinolon-16,21-diacetat, Triamcinolonacetonid-21-acetat, Triamcinolonacetonid-21-dinatriumphosphat, Triamcinolonacetonid-21-hemisuccinat, Mometasonfuroat oder Loteprednol-etabonat, oder eine Kombination davon, wie beispielsweise Ipratropiumbromid in Kombination mit Salbutamolsulfat.

Gemäss einem weiteren bevorzugten Aspekt können die erfindungsgemäss erhältlichen Formulierungen insbesondere auch ein Corticosteroid, wie Ciclosenid, Rofleponid, Fluticason-propionat, Mometasonfuroat oder Loteprednol-etabonat, in Kombination mit einem Betamimetikum, wie Formoterolfumarat, Formoteroltartrat, Levalbuterolsulfat oder Salmeterolxinafoat, enthalten.

Die Wirkstoffmenge in den Formulierungen kann in breiten Bereichen variieren und ist in hohem Masse vom jeweiligen Wirkstoff und bis zu einem gewissen Grad auch vom verwendeten Pulverinhalator abhängig. Typischerweise kann die Wirkstoffkonzentration etwa 0,1 bis 10 Gew.-%, insbesondere etwa 0,1 bis 5 Gew.-%, bezogen auf die Gesamtformulierung, betragen. Gelegentlich können auch höhere oder tiefere Konzentrationen zweckmässig sein, wobei aber Wirkstoffkonzentrationen unter 0,001 Gew.-% oder unter 0,01 Gew.-% selten vorkommen.

Zur exakten volumetrischen Dosierung der meisten Wirkstoffe bzw. Formulierungen ist eine Verdünnung des Wirkstoffes mit einem pharmazeutisch inaktiven Hilfsstoff erforderlich, um eine den Anforderungen an die Dosiergenauigkeit genügende, dosierbare Einheitsmenge zu erhalten. Hierzu werden die mikrofeinen, inhalierbaren Wirkstoffpartikel mit pharmakologisch inaktiven Substanzen (Trägern) gemischt. Die Verdünnung wird dabei so gewählt, dass die vom Pulverinhalator ausgebrachte Menge genau die gewünschte Dosis enthält. Der pharmakologisch inaktive Hilfsstoff dient vorzugsweise nicht nur zum Verdünnen, sondern auch zum Einstellen einer möglichst guten Fliessfähigkeit der Pulvermischung, und er ist im Falle der bevorzugt verwendeten sogenannten interaktiven oder geordneten Mischungen die Trägersubstanz, an die die mikrofeinen Wirkstoffpartikel durch Adhäsion gebunden werden, um so eine geeignete Mischgüte, d.h. Homogenität der Mischung, zu erreichen und aufrecht zu erhalten.

Der Träger liegt in der Formulierung vorzugsweise in einer Partikelgrösse vor, die nicht inhalierbar ist. Die Trägerteilchen sollten aber andererseits nicht zu gross sein, da sich dies nachteilig auf die FPF auswirken kann. Die optimale Partikelgrösse des eingesetzten Trägers richtet sich dabei in der Regel nach den Anforderungen und Gegebenheiten des Pulverinhalators, der für die Applikation der Formulierung vorgesehen ist. Im Rahmen der vorliegenden Erfindung können Träger mit üblichen Teilchengrössen verwendet werden, und optimale Teilchengrössen können vom Fachmann von Fall zu Fall leicht ermittelt werden. Im allgemeinen kann jedoch der mittlere Teilchendurchmesser (MMAD) der Trägerpartikel etwa 10 bis 500 µm und vorzugsweise etwa 50 bis 200 µm betragen.

Die Haftung der Wirkstoffpartikel an den Trägerteilchen sollte ausreichend sein, dass während den Verarbeitungs-, Transport-, Lager- und Dosiervorgängen keine Entmischung stattfindet, aber andererseits nicht so hoch, dass eine möglichst quantitative Ablösung der Wirkstoffteilchen während dem Dispergieren im Inhalator, ausgelöst durch den Atemfluss des Patienten, nicht mehr gewährleistet ist. Die Effektivität der Freisetzung der Wirkstoffpartikel ist neben den physikalisch-chemischen Eigenschaften des Wirkstoffes und den aerodynamischen Eigenschaften des Pulverinhalators vor allem von den Eigenschaften des Trägers, insbesondere der Art des Trägers und dessen Oberflächenstruktur, mittlerer Partikelgrösse und Partikelgrössenverteilung, abhängig.

Im Rahmen der vorliegenden Erfindung sind grundsätzlich alle üblicherweise in Trockenpulver-Formulierungen verwendeten Trägermaterialien geeignet, beispielsweise Mono- oder Disaccharide, wie Glucose, Lactose, Lactose-Monohydrat, Saccharose oder Trehalose, Zuckeralkohole, wie Mannitol oder Xylitol, Polymilchsäure oder Cyclodextrin, wobei Glucose, Trehalose und insbesondere Lactose-Monohydrat im allgemeinen bevorzugt sind. Gewünschtenfalls können die Formulierungen auch zwei oder mehrere Trägermaterialien enthalten. Gewünschtenfalls kann die Formulierung neben nicht inhalierbaren Trägerpartikeln auch einen Anteil an inhalierbaren Trägerpartikeln enthalten; zum Beispiel kann sie neben gröberen Lactose-Monohydrat-Trägerpartikeln einen Anteil von z.B. 0,1 bis 10 Gew.-% an mikronisiertem Lactose-Monohydrat enthalten, das beispielsweise für mindestens 50% der Partikel einen Teilchendurchmesser von höchstens 10 µm, vorzugsweise höchstens 5 µm, aufweisen kann.

Der Anteil an Trägermaterial in den erfindungsgemäss erhältlichen Formulierungen kann je nach der für den jeweiligen Wirkstoff nötigen oder wünschbaren Verdünnung und der zur Verbesserung der Feuchtigkeitsbeständigkeit verwendeten Menge an Magnesiumstearat in einem breiten Bereich variieren. Der Anteil an Trägermaterial in der Gesamtformulierung kann beispielsweise etwa 80 bis 99,9 Gew.-% betragen, wobei aber je nach Wirkstoff auch höhere oder niedrigere Anteile vorteilhaft sein können.

Die Konzentration an Magnesiumstearat kann von etwa 0,4 bis 0,8 Gew.-%, vorzugsweise etwa 0,5 bis 0,75 Gew. -%, bezogen auf die Gesamtformulierung, betragen.

Die Formulierungen können gewünschtenfalls weitere Komponenten enthalten. Vorzugsweise bestehen sie jedoch aus einem oder mehreren pharmazeutisch nicht wirksamen Trägern, einem oder mehreren pharmazeutischen Wirkstoffen und Magnesiumstearat.

Die Trockenpulver-Formulierungen können dadurch hergestellt werden, dass man einen pharmazeutisch nicht wirksamen Träger in nicht inhalierbarer Teilchengrösse (der gewünschtenfalls einen Anteil in inhalierbarer Teilchengrösse enthalten kann), einen fein verteilten pharmazeutischen Wirkstoff in inhalierbarer Teilchengrösse, beispielsweise mit einem mittleren Teilchendurchmesser von höchstens 10 µm (vorzugsweise höchstens 5 µm), und Magnesiumstearat miteinander vermischt. Die Bestandteile können grundsätzlich in beliebiger Reihenfolge miteinander gemischt werden, wobei aber die Vermischung zweckmässigerweise so erfolgen sollte, dass die Partikel der Bestandteile - abgesehen von der Adhäsion an die Trägerpartikel - im wesentlichen als solche erhalten bleiben, d.h. nicht z.B. durch eine Granulierung und dergleichen zerstört werden. Gemäss einer bevorzugten Variante kann jedoch zuerst eine Vormischung von Magnesiumstearat mit dem Träger hergestellt und dann die Wirkstoffpartikel zugemischt werden. Gemäss einer weiteren bevorzugten Variante kann zuerst eine Vormischung des Wirkstoffes mit dem Träger hergestellt und dann das Magnesiumstearat zugemischt werden. Das Mischen kann in an sich bekannter Weise, beispielsweise in einem Taumelmischer, erfolgen. Vorzugsweise kann in diesen Verfahren jeweils staubförmiges Magnesiumstearat mit einer mittleren Teilchengrösse und etwa 1 bis 100 µm, insbesondere etwa 5 bis 20 µm, zugesetzt werden.

Die beschriebenen Trockenpulver-Formulierungen werden in Multidosis-Trockenpulverinhalatoren, die ein Pulverreservoir enthalten, insbesondere in Multidosis-Pulverinhalatoren wie in WO-A-97/20589 beschrieben, angewendet.

Spezielle, erfindungsgemäss in den Multidosis-Trockenpulverinhalator enthaltene Trockenpulver-Formulierungen zur Inhalation mit verbesserter Feuchtigkeitsbeständigkeit umfassen einen pharmazeutisch nicht wirksamen Träger in nicht inhalierbarer Teilchengrösse, einen fein verteilten pharmazeutischen Wirkstoff in Form eines pharmazeutisch annehmbaren Salzes oder Esters in inhalierbarer Teilchengrösse (vorzugsweise mit einem mittleren Teilchendurchmesser von höchstens 10 µm, insbesondere höchstens 5 µm) und 0,4 bis 1 Gew.-%, bezogen auf die Gesamtformulierung, an Magnesiumstearat. Bevorzugt sind Trockenpulver-Formulierungen, die in Form von interaktiven Mischungen vorliegen. Bevorzugte Wirkstoffsalze und -ester, Trägermaterialien, Mengenbereiche, Methoden und dergleichen ergeben sich aus der vorangehenden Beschreibung.

Die Erfindung wird durch die folgenden Beispiele weiter veranschaulicht. In den Beispielen bezeichnet r.F. die relative Luftfeuchtigkeit; die Bezeichnung n.b. gibt an, dass der betreffende Wert nicht bestimmt wurde. Die Tests erfolgten jeweils mit einem Trockenpulverinhalator vom Typ SkyePharma mDPI (SkyePharma AG, Schweiz) gemäss WO-A-97/20589. Die Bestimmung der FPD und der FPF erfolgte - sofern nicht anders angegeben - jeweils mit einem Twin Impinger. Siebungen erfolgten - sofern nicht anders angegeben - jeweils mit einem Sieb mit einer Lochweite von 180 µm. Zur Ermittlung der Feuchtigkeitsempfindlichkeit wurden die Trockenpulver, ausser im Beispiel 7, jeweils offen ohne Feuchtigkeitsschutz gelagert.

### Beispiel 1

198,46 g Lactose-Monohydrat mit einer definierten Korngrösse < 200 µm für 100%, < 125 µm für 50% und < 75 µm für 10% der Partikel(Siebanalyse) werden gesiebt und mit 1 g gesiebtem Magnesiumstearat mit einem Taumelmischer gemischt. Im Anschluss werden 0,54 g Formoterolfumarat-Dihydrat und die Vormischung gesiebt und gemischt. Die so erhaltene Mischung wird in einen geeigneten dosierenden Trockenpulverinhalator gefüllt. Für die genaue analytische Bestimmung der Partikelgrössenverteilung und speziell der FPD bzw. FPF wird eine adäquate Anzahl Dosierungen in einen im Europäischen Arzneibuch oder anderen nationalen Pharmakopöen beschriebenen Impaktor oder Impinger, z.B. den sogenannten Twin Impinger oder Multi Stage Liquid Impinger, nach ebenfalls dort beschriebenen Verfahren abgegeben und gesammelt. Die aufgefangenen und abgeschiedenen Wirkstoffpartikel werden in analytischen Standardverfahren zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40°C / 75% r.F. oder einer anderen geeigneten Bedingung über einen Zeitraum von mehreren Tagen bis Wochen gelagert und anschliessend wie oben beschrieben im Pulverinhalator getestet.

Die mit dem hergestellten ternären Gemisch (Formulierung 1-A) und mit konventionellen Mischungen (Formulierungen 1-B und 1-C) in einem 5-Stufen Liquid Impinger gemäss Ph. Eur. erhaltenen Ergebnisse sowie die Zusammensetzungen der Mischungen (in Gew.-%) sind in Tabelle 1 aufgeführt. Im Vergleich zu den konventionellen interaktiven Mischungen zeigt die erfindungsgemässe ternäre Mischung mit Magnesiumstearat den Vorteil einer erhöhten FPD bzw. FPF und einer wesentlich verbesserten Stabilität der FPD bzw. FPF bei Lagerung bei 40°C / 75% r.F. Wie die Ergebnisse zu Formulierung 1-C zeigen, kann in konventionellen Formulierungen durch Zusatz von mikronisierter Lactose zwar eine anfängliche Erhöhung der FPD und FPF erreicht werden, nicht aber ein Schutz gegen den Einfluss erhöhter Temperatur und Feuchte. Dies ist ebenfalls aus den für die Formulierungen 1-A und 1-C unmittelbar nach Herstellung bzw. nach 7 oder 13 Tagen Lagerung des Trockenpulvers bei 40°C / 75% r.F. ermittelten MMAD-Werten ersichtlich: für Formulierung 1-A nach Herstellung 1,8 µm, nach 7 Tagen 1,9 µm und nach 13 Tagen 1,9 µm; für Formulierung 1-C nach Herstellung 2,2 µm, nach 7 Tagen 4,5 µm und nach 13 Tagen 5,5 µm. Im Gegensatz zur konventionellen Formulierung bleibt somit der MMAD bei der erfindungsgemässen Formulierung konstant, was die Ergebnisse der FPD- und FPF-Untersuchung bestätigt.

**Tabelle 1**

| Formulierung | 1-A | 1-B (Vergleich) | 1-C (Vergleich) |
|---|---|---|---|
| Lactose-Monohydrat | 99, 23 % | 99, 73 % | 97,24 % |
| Lactose-Monohydrat mikronisiert | 0,00 % | 0,00 % | 2,49 % |
| Magnesiumstearat | 0,50 % | 0,00 % | 0,00 % |
| Formoterolfumarat-Dihydrat mikronisiert | 0,27 % | 0,27 % | 0,27 % |
| FPD nach Herstellung [µg pro Hub] | 4,7 | 1,3 | 3,3 |
| FPD nach 3-4 Tagen bei 40°C/75% r.F. [µg pro Hub] | 4,5 | n.b. | 1,0 |
| FPF nach Herstellung [% gefundener Wirkstoff] | 42,5 | 13,7 | 35,9 |
| FPF nach 3-4 Tagen bei 40°C/75% r.F. [% gefundener Wirkstoff] | 37,3 | n.b. | 11,0 |

### Beispiel 2

97,23 g Lactose-Monohydrat mit einer definierten Korngrösse < 200 µm für 100%, < 125 µm für 50% und < 75 µm für 10% der Partikel (Siebanalyse) werden gesiebt und mit 2,5 g gesiebtem mikronisiertem Lactose-Monohydrat (50% der Partikel < 5 µm) in einem Taumelmischer gemischt. Im Anschluss werden 0,27 g Formoterolfumarat-Dihydrat und die Vormischung gesiebt und gemischt. Die so erhaltene Mischung wird mit 0,125 g gesiebtem Magnesiumstearat gemischt und in einen geeigneten dosierenden Trockenpulverinhalator gefüllt. Für die analytische Bestimmung der FPD bzw. FPF wird eine adäquate Anzahl Dosierungen in einen Twin Impinger oder Multi Stage Liquid Impinger abgegeben und gesammelt. Die aufgefangenen und abgeschiedenen Wirkstoffpartikel werden zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40°C / 75% r.F. über einen Zeitraum von einigen Tagen gelagert und anschliessend wie oben beschrieben im Pulverinhalator getestet.

Die mit dem herstellten Gemisch (Formulierung 2) und mit einer konventionellen Mischung (Formulierung 1-C) in einem 5-Stufen Liquid Impinger gemäss Ph. Eur. erhaltenen Ergebnisse sowie die Zusammensetzungen der Mischungen (in Gew.-%) sind in Tabelle 2 aufgeführt. Im Vergleich zur konventionellen interaktiven Mischung zeigt die erfindungsgemässe Mischung mit Magnesiumstearat den Vorteil einer erhöhten FPD bzw. FPF und einer verbesserten Stabilität der FPD bzw. FPF bei Lagerung bei 40°C / 75% r.F.

**Tabelle 2**

| Formulierung | 2 | 1-C (Vergleich) |
|---|---|---|
| Lactose-Monohydrat | 96,75 % | 97,24 % |
| Lactose-Monohydrat mikronisiert | 2,48 % | 2,49 % |
| Magnesiumstearat | 0,50 % | 0,00 % |
| Formoterolfumarat-Dihydrat mikronisiert | 0,27 % | 0,27 |
| FPD nach Herstellung [µg pro Hub] | 5,3 | 3,3 |
| FPD nach 3-4 Tagen bei 40°C/75% r.F. [µg pro Hub] | n.b. | 1,0 |
| FPF nach Herstellung [% gefundener Wirkstoff] | 41,4 | 35,9 |
| FPF nach 3-4 Tagen bei 40°C/75% r.F. [% gefundener Wirkstoff] | n.b. | 11,0 |

### Beispiel 3

97 g Lactose-Monohydrat mit einer definierten Korngrösse < 110 µm für 90%, < 70 µm für 50% und < 40 µm für 10% der Partikel (Siebanalyse) werden gesiebt und mit 0,5 g gesiebtem Magnesiumstearat in einem Taumelmischer gemischt. Im Anschluss werden 2,5 g Salbutamolsulfat und die Vormischung gesiebt und gemischt. Die so erhaltene Mischung wird in einen geeigneten dosierenden Trockenpulverinhalator gefüllt. Für die analytische Bestimmung der FPD bzw. FPF wird eine adäquate Anzahl Dosierungen in einen Twin Impinger abgegeben und gesammelt. Die aufgefangenen und abgeschiedenen Wirkstoffpartikel werden zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40 °C / 75 % r.F. über einen Zeitraum von 7 Tagen gelagert und anschliessend wie oben beschrieben im Pulverinhalator getestet.

Die mit dem herstellten ternären Gemisch (Formulierung 3-A) und mit einer konventionellen binären Mischung (Formulierung 3-B) in einem Twin Impinger gemäss Ph. Eur. erhaltenen Ergebnisse sowie die Zusammensetzungen der Mischungen (in Gew.-%) sind in Tabelle 3 aufgeführt. Die ternäre Mischung mit Magnesiumstearat erzielt eine höhere FPD bzw. FPF und ist wesentlich stabiler bei Lagerung bei 40°C / 75% r.F.

**Tabelle 3**

| Formulierung | 3-A | 3-B (Vergleich) |
|---|---|---|
| Lactose-Monohydrat | 97,00 % | 97,50 % |
| Magnesiumstearat | 0,50 % | 0,00 % |
| Salbutamolsulfat mikronisiert | 2,50 % | 2,50 % |
| FPD nach Herstellung [µg pro Hub] | 39,5 | 26,2 |
| FPD nach 7 Tagen bei 40°C/75% r.F. [µg pro Hub] | 27,8 | 11,3 |
| FPF nach Herstellung [% gefundener Wirkstoff] | 37,4 | 25,3 |
| FPF nach 7 Tagen bei 40°C/75% r.F. [% gefundener Wirkstoff] | 35,6 | 9,7 |

### Beispiel 4

1196 g Lactose-Monohydrat mit einer definierten Korngrösse < 315 µm für 100%, < 150 µm für 55-90% und < 63 µm für maximal 10% der Partikel (Siebanalyse) werden gesiebt und mit 3 g gesiebtem Magnesiumsstearat in einem Taumelmischer (tumble blender TB) gemischt. Im Anschluss werden 1,44 g Formoterolfumarat-Dihydrat und die Vormischung gesiebt und gemischt. In analoger Weise werden unter Variation der Ansatzgrösse, der Prozessparameter und der Magnesiumstearat- und Formoterolfumarat-Mengen weitere Formulierungen hergestellt, um deren Einfluss auf die Stabilität der FPD zu untersuchen. Die erhaltenen Mischungen werden - nach Herstellung oder nach anschliessender Lagerung der offenen Mischung bei erhöhter Temperatur und Feuchtigkeit - in einen geeigneten dosierenden Trockenpulverinhalator gefüllt. Die in-vitro Partikelgrössenverteilung und die FPD bzw. FPF werden mit einem Multi-Stage Liquid Impinger an einer adäquaten Anzahl Dosierungen bestimmt.

Die Ergebnisse zeigten, dass bei Herstellung der Pulvermischungen mit einem Taumelmischer praktisch nur die Konzentration von Magnesiumstearat für die Stablilität hinsichtlich der FPD verantwortlich ist, während die anderen Parameter im untersuchten Bereich praktisch ohne Bedeutung für die Stabilität bei erhöhter Feuchtigkeit waren. In Tabelle 4 sind die Ansatzgrösse, die Konzentration an Magnesiumstearat (MS) und die Konzentration an Formoterolfumarat-Dihydrat (FF) für einige repräsentative Mischungen sowie deren in einem 5-Stufen Liquid Impinger gemäss Ph. Eur. gemessene FPF-Werte, die unmittelbar nach Herstellung bzw. nach Lagerung bei 40°C / 75% r.F. während 7 Tagen erhalten wurden, zusammengestellt. Die angegebenen Werte sind Mittelwerte aus je drei Bestimmungen. Die Ergebnisse zeigen, dass die FPF bei ausreichender Magnesiumstearatkonzentration durch erhöhte Temperatur und Feuchtigkeit kaum mehr beeinträchtigt wird. Die für Formulierung 1-A nach 3 Wochen Lagerung bei 40°C / 75% r.F. gemessene FPF von 32,3% scheint zudem anzudeuten, dass selbst bei suboptimaler Magnesiumstearatkonzentration ein langanhaltender Schutz gegen den Einfluss erhöhter Temperatur und Feuchtigkeit erreicht wird.

**Tabelle 4**

| Formulierung | Ansatzgrösse | MS | FF | FPF nach 0 d | FPF nach 7 d |
|---|---|---|---|---|---|
| | [kg] | [%G/G] | [%G/G] | [%] | [%] |
| 4-A | 1,2 | 0,25 | 0,12 | 42,5 | 33,6 |
| 4-B | 4,8 | 0,50 | 0,12 | 49,3 | n.b. |
| 4-C | 4,8 | 0,75 | 0,12 | 56,9 | 56,8 |
| 4-D | 1,2 | 0,25 | 0,34 | 50,0 | 33,5 |
| 4-E | 4,8 | 0,50 | 0,34 | 58,1 | n.b. |
| 4-F | 4,8 | 0,75 | 0,34 | 59,2 | 57,2 |
| 1-C (Vergleich) | 0,2 | 0,00 | 0,27 | 39,7 | 12,3 |
| 1-A | 0,2 | 0,50 | 0,27 | 44,8 | 32,5 |

### Beispiel 5

49,5 g Lactose-Monohydrat mit einer definierten Korngrösse < 200 µm für 100%, < 125 µm für 50% und < 75 µm für 10% der Partikel (Siebanalyse) werden gesiebt und mit 0,25 g gesiebtem Magnesiumstearat in einem Taumelmischer gemischt. Im Anschluss werden 0,25 g Salbutamolsulfat und die Vormischung gesiebt und gemischt. In analoger Weise werden unter Variation der Konzentration an Magnesiumstearat (MS) und Salbutamolsulfat (SS) weitere Mischungen gemäss Tabelle 5 hergestellt. Die erhaltenen Mischungen werden unmittelbar nach Herstellung oder nach Lagerung bei 40°C / 75% r.F. während 5 bzw. 21 Tagen in einen geeigneten dosierenden Trockenpulverinhalator gefüllt. Zur Bestimmung der FPD bzw. FPF wird eine adäquate Anzahl Dosierungen in einen Twin Impinger gemäss Ph. Eur. abgegeben, gesammelt und der Wirkstoffgehalt der einzelnen Fraktionen analytisch bestimmt.

Die in Tabelle 5 angegebenen FPF-Werte (Mittelwerte aus zwei Messungen) zeigen, dass Magnesiumstearat auch im Falle des feuchtigkeitsempfindlichen Wirkstoffes Salbutamolsulfat einen Schutz gegen erhöhte Temperatur und Feuchtigkeit bewirkt, aber eine Stabilisierung der FPF erst bei höheren Magnesiumstearat-Konzentrationen erreicht wird als bei den Formoterolfumarat-Zubereitungen.

**Tabelle 5**

| Formulierung | MS [%G/G] | SS [%G/G] | FPF [%] nach | | |
|---|---|---|---|---|---|
| | | | 0 d | 5 d | 21 d |
| 5-A | 0,5 | 0,5 | 9,3 | 14,2 | 12,0 |
| 5-B | 0,5 | 1,0 | 22,3 | 17,1 | 14,9 |
| 5-C | 0,5 | 2,5 | 30,2 | 25,6 | 22,3 |
| 5-D | 1,0 | 0,5 | 19,0 | 18,8 | 13,5 |
| 5-E | 1,0 | 1,0 | 23,0 | 20,1 | 15,8 |
| 5-F | 1,0 | 2,5 | 25,0 | 22,6 | 20,8 |
| 5-G | 2,5 | 1,0 | 22,7 | 23,4 | 21,5 |
| 5-H | 2,5 | 2,5 | 25,9 | 26,4 | 27,4 |
| Vergleich: | | | | | |
| 5-I | 0,0 | 0,5 | 13,5 | 5,3 | 4,0 |
| 5-J | 0,0 | 1,0 | 19,7 | 9,5 | 6, 6 |
| 5-K | 0,0 | 2,5 | 25,3 | 14,8 | 13,9 |

### Beispiel 6

99,2 g Lactose-Monohydrat mit einer Korngrösse < 315 µm für 100%, < 150 µm für 55-90% und < 63 µm für maximal 10% der Partikel (Siebanalyse) werden gesiebt und mit 0,5 g gesiebtem Magnesiumstearat in einem Taumelmischer gemischt. Im Anschluss werden 0,34 g Tiotropiumbromid und die Vormischung gesiebt und gemischt. Die erhaltene Mischung wird nach der Herstellung bzw. nach Lagerung bei 40°C / 75% r.F. während 7 Tagen in einen geeigneten dosierenden Trockenpulverinhalator gefüllt. Zur Bestimmung der FPD bzw. FPF wird eine adäquate Anzahl Dosierungen in einen Multi-Stage Impinger gemäss Ph. Eur. abgegeben, gesammelt und der Wirkstoffgehalt der einzelnen Fraktionen analytisch bestimmt. Die unmittelbar nach Herstellung abgefüllten Proben ergaben eine FPD von 8,0 µg und eine FPF von 48,4% (Mittelwerte aus 2 Messungen); für die während 7 Tagen unter feuchten Bedingungen gelagerten Proben wurden eine FPD von 6,9 µg und eine FPF von 43,0% erhalten (Mittelwerte aus 4 Messungen), d.h. die Stabilisierung mit 0,5% Magnesiumstearat ergibt auch im Falle des feuchtigkeitsempfindlichen Tiotropiumbromids eine hinreichend gleichmässige FPD bzw. FPF.

### Beispiel 7

Zur Untersuchung des Einflusses erhöhter Feuchtigkeit und Temperatur auf erfindungsgemässe Formulierungen unter praxisnahen Bedingungen wurden Trockenpulverinhalatoren vom Typ SkyePharma mDPI (SkyePharma AG, Schweiz), entsprechend der Offenbarung von WO-A-97/20589, mit je 2 g gemäss Beispiel 1 frisch hergestelltem Trockenpulver aus 99,23 Gew.-% Lactose-Monohydrat, 0,50 Gew.-% Magnesiumstearat und 0,27 Gew.-% mikronisiertem Formoterolfumarat-Dihydrat (Formulierung 1-A) gefüllt. Die in-vitro-Daten wurden unmittelbar nach dem Füllen sowie nach 3, 6 und 12 Monaten Lagerung der unverpackten Inhalatoren ohne Feuchtigkeitsschutz bei verschiedenen Temperatur- und Feuchtebedingungen ermittelt. Die abgegebenen Dosen und die Hubmassen wurden anhand der Hübe Nr. 2-4, 149-152 und 298-300 aus je drei Inhalatoren bestimmt, die gemäss der von Collins auf der Konferenz Drug Delivery to the Lungs VIII, London, Dezember 1998 (Tagungsunterlagen Seiten 116-119) beschriebenen Methode in einen Buchner-Trichter abgegeben wurden. Die FPD bzw. FPF wurde bei 60 l/min. mittels eines 5-Stufen Liquid Impactors gemäss Ph. Eur. anhand der Hübe Nr. 6-15 und 287-296 aus je drei Inhalatoren bestimmt. Die in Tabelle 6 zusammengestellten Mittelwerte und relativen Standardabweichungen zeigen, dass die erfindungsgemässe Formulierung selbst bei vergleichsweise extremen Temperatur- und Feuchtebedingungen über lange Zeiträume kaum beeinträchtigt wird.

**Tabelle 6**

| Lagerung | | Hubmasse [mg] | Abgegebene Dosis [µg] | FPF [%] | FPD [µg] |
|---|---|---|---|---|---|
| nein | | 6, 0 (± 5,4%) | 10,2 (± 10,1%) | 43,5 | 6,0 |
| 25°C / 60% r.F.: | | | | | |
| 3 | Monate | 6,1 (± 4,8%) | 10,5 (± 9,5%) | 40,8 | 5,4 |
| 6 | Monate | 5,9 (± 8,2%) | 10,9 (± 6,9%) | 47,8 | 7,0 |
| 12 | Monate | 6,1 (± 5,0%) | 12,1 (± 5,9%) | 42,2 | 5,9 |
| 30°C / 70% r.F.: | | | | | |
| 3 | Monate | 6,1 (± 6,9%) | 11,0 (± 12,9%) | 40,1 | 5, 6 |
| 6 | Monate | 6,2 (± 8,7%) | 10,6 (± 11,5%) | 39,9 | 5,7 |
| 12 | Monate | 6,3 (± 4,3%) | 10,7 (± 5,9%) | 42,0 | 5,7 |
| 40°C / 75% r.F.: | | | | | |
| 3 | Monate | 5,8 (± 9,7%) | 9,9 (± 9,8%) | 38,1 | 5,2 |
| 6 | Monate | 6,0 (± 19,5%) | 10,3 (± 19,2%) | 35,1 | 4,9 |
| 12 | Monate | 6,7 (± 6,8%) | 10,7 (± 7,9%) | 37,9 | 5,4 |

### Beispiel 8

In analoger Weise zu Beispiel 4 wurde ein Trockenpulver, bestehend aus 0,2 Gew.-% Formoterolfumarat-Dihydrat, 0,5 Gew.-% Glycopyrrolate, 0,5 Gew.-% Magnesiumstearat und 98,8 Gew.-% Lactose-Monohydrat hergestellt.

## Patentansprüche

1. Verwendung von staubförmigem Magnesiumstearat bei der Herstellung von einem Multidosis-Inhalator, der eine vordosierte Einheit enthaltend eine Trockenpulver-Formulierung zur Inhalation umfasst, zur Verbesserung des Beständigkeit der Trockenpulver-Formulierung gegen Feuchtigkeit, worin die Trockenpulver-Formulierung einen pharmazeutisch nicht wirksamen Träger in nicht inhalierbarer Teilchengrösse, einen fein verteilten pharmazeutischen Wirkstoff in der Form von Partikeln in inhalierbarer Teilchengrösse und 0,4 bis 1 Gew-% Magnesiumstearat, bezogen auf die Trockenpulverformulierung, umfasst.

2. Verwendung von staubförmigem Magnesiumstearat bei der Herstellung von einem Multidosis-Inhalator, der eine vordosierte Einheit enthaltend eine Trockenpulver-Formulierung zur Inhalation umfasst, zur Verringerung des Einflusses von eindringender Feuchtigkeit auf deren Feinpartikelanteil (FPF), worin die Trockenpulver-Formulierung einen pharmazeutisch nicht wirksamen Träger in nicht inhalierbarer Teilchengrösse, einen fein verteilten pharmazeutischen Wirkstoff in der Form von Partikeln in inhalierbarer Teilchengrösse und 0,4 bis 1 Gew-% Magnesiumstearat, bezogen auf die Trockenpulverformulierung, umfasst.

3. Verwendung von Magnesiumstearat gemäss Anspruch 1 oder 2, **dadurch gekennzeichnet, dass** der pharmazeutisch nicht wirksame Träger einen mittleren Teilchendurchmesser (MMAD) von etwa 10 bis 500 µm hat und der fein verteilte pharmazeutische Wirkstoff in der Form von Partikeln mit einem mittleren Teilchendurchmesser (gemessen als MMAD) von höchstens etwa 10 µm ist.

4. Verwendung gemäss irgendeinem der Ansprüche 1 bis 3, **dadurch gekennzeichnet, dass** der pharmazeutisch nicht wirksame Träger, der fein verteilte pharmazeutische Wirkstoff und das Magnesiumstearat in Form einer interaktiven Mischung vorliegen.

5. Verwendung gemäss irgendeinem der Ansprüche 1 bis 4, **dadurch gekennzeichnet, dass** die Trockenpulverformulierung erhältlich ist, indem Lactose-Monohydrat mit einem mittleren Teilchendurchmesser (MMAD) von etwa 10 bis 500 µm als ein Träger, der fein verteilte pharmazeutische Wirkstoff mit einem mittleren Teilchendurchmesser (gemessen als MMAD) von höchstens 10 µm und das staubförmige Magnesiumstearat so gemischt werden, dass die Partikel der besagten Bestandteile - abgesehen von der Adhäsion an die Trägerpartikel - als solche erhalten bleiben.

6. Verwendung gemäss einem der Ansprüche 1 bis 5, **dadurch gekennzeichnet, dass** das staubförmige Magnesiumstearat in der Trockenpulver-Formulierung in einer Konzentration von 0,4 bis 0,8 Gew.-%, vorzugsweise 0,5 bis 0,75 Gew.-%, vorliegt.

7. Verwendung gemäss irgendeinem der Ansprüche 1 bis 6, **dadurch gekennzeichnet, dass** der pharmazeutische Wirkstoff ein Betamimetikum und/oder ein Anticholinergikum und/oder ein Corticosteroid oder ein pharmazeutisch annehmbares Salz oder Ester davon ist.

8. Verwendung gemäss Anspruch 7, worin das Corticosteroid Fluticason ist.

9. Verwendung gemäss irgendeinem der Ansprüche 1 bis 8, **dadurch gekennzeichnet, dass** die Trockenpulver-Formulierung als pharmazeutischen Wirkstoff ein Corticosteroid in Kombination mit einem Betamimetikum, vorzugsweise ein aus der Gruppe bestehend aus Ciclosenid, Rofleponid, Fluticason-propionat, Mometasonfuroat und Loteprednol-etabonat ausgewähltes Corticosteroid in Kombination mit einem aus der Gruppe bestehend aus Formoterolfumarat, Formoteroltartrat, Levalbuterolsulfat und Salmeterolxinafoat ausgewählten Betamimetikum, umfasst.

10. Verwendung gemäss einem der Ansprüche 1 bis 9, **dadurch gekennzeichnet, dass** die Trockenpulver-Formulierung neben den nicht inhalierbaren Trägerpartikeln auch einen Anteil an inhalierbaren Trägerpartikeln enthält; insbesondere neben nicht inhalierbaren Lactose-Monohydrat-Trägerpartikeln auch einen Anteil von 0,1 bis 10 Gew.-% an mikronisiertem Lactose-Monohydrat enthält.

## Claims

1. Use, to improve the resistance of a dry powder formulation to moisture, of magnesium stearate in powder form in the production of a multi-dose inhaler having a pre-dosed unit containing a dry powder formulation for inhalation, wherein the dry powder formulation comprises a pharmaceutically inactive carrier in a non-inhalable particle size, a finely distributed pharmaceutical active substance in the form of particles in an inhalable particle size and 0.4 to 1 wt.%, based on the dry powder formulation, of magnesium stearate.

2. Use, to reduce the effect of penetrating moisture on the fine particle fraction (FPF) of a dry powder formulation, of magnesium stearate in powder form in the production of a multi-dose inhaler having a pre-dosed unit containing a dry powder formulation for inhalation, wherein the dry powder formulation comprises a pharmaceutically inactive carrier in a non-inhalable particle size, a finely distributed pharmaceutical active substance in the form of particles in an inhalable particle size and 0.4 to 1 wt.%, based on the dry powder formulation, of magnesium stearate.

3. Use of magnesium stearate according to claim 1 or 2, **characterised in that** the pharmaceutically inactive carrier has an average particle diameter (MMAD) of approximately 10 to 500 µm and the finely distributed pharmaceutical active substance in the form of particles has an average particle diameter (measured as MMAD) of at most approximately 10 µm.

4. Use according to one of claims 1 to 3, **characterised in that** the pharmaceutically inactive carrier, the finely distributed pharmaceutical active substance and the magnesium stearate are present in the form of an interactive mixture.

5. Use according to one of claims 1 to 4, **characterised in that** the dry powder formulation can be obtained by mixing lactose monohydrate having an average particle diameter (MMAD) of approximately 10 to 500 µm as a carrier, the finely distributed pharmaceutical active substance having an average particle diameter (measured as MMAD) of at most 10 µm and the magnesium stearate in powder form in such a way that the particles of said constituents remain as such, aside from the adhesion to the carrier particles.

6. Use according to one of claims 1 to 5, **characterised in that** the magnesium stearate in powder form in the dry powder formulation is present in a concentration of 0.4 to 0.8 wt.%, preferably 0.5 to 0.75 wt.%.

7. Use according to one of claims 1 to 6, **characterised in that** the pharmaceutical active substance is a beta mimetic and/or an anticholinergic and/or a corticosteroid or a pharmaceutically acceptable salt or ester.

8. Use according to claim 7, wherein the corticosteroid is fluticasone.

9. Use according to one of claims 1 to 8, **characterised in that** the dry powder formulation comprises a corticosteroid in combination with a beta mimetic as the pharmaceutical active substance, preferably a corticosteroid selected from the group consisting of ciclesonide, rofleponide, fluticasone propionate, mometasone furoate and loteprednol etabonate in combination with a beta mimetic selected from the group consisting of formoterol fumarate, formoterol tartrate, levalbuterol sulphate and salmeterol xinafoate.

10. Use according to one of claims 1 to 9, **characterised in that** the dry powder formulation also contains a proportion of inhalable carrier particles in addition to the non-inhalable carrier particles, in particular a proportion of 0.1 to 10 wt.% of micronised lactose monohydrate in addition to non-inhalable lactose monohydrate carrier particles.

## Revendications

1. Utilisation, pour améliorer la résistance d'une formulation de poudre sèche à l'humidité, de stéarate de magnésium sous forme de poudre dans la production d'un inhalateur multidose comportant une unité prédosée contenant une formulation de poudre sèche pour inhalation, dans laquelle la formulation de poudre sèche comprend un véhicule pharmaceutiquement inactif ayant une taille de particule ne permettant pas l'inhalation, une substance active pharmaceutique finement répartie sous forme de particules ayant une taille de particule permettant l'inhalation, et 0,4 à 1 % en poids, relativement à la formulation de poudre sèche, de stéarate de magnésium.

2. Utilisation, pour réduire l'effet de l'humidité pénétrante sur la fraction granulométrique fine (FGF) d'une formulation de poudre sèche, de stéarate de magnésium sous forme de poudre dans la production d'un inhalateur multidose comportant une unité prédosée contenant une formulation de poudre sèche pour inhalation, dans laquelle la formulation de poudre sèche comprend un véhicule pharmaceutiquement inactif ayant une taille de particule ne permettant pas l'inhalation, une substance active pharmaceutique finement répartie sous forme de particules ayant une taille de particule permettant l'inhalation, et 0,4 à 1 % en poids, relativement à la formulation de poudre sèche, de stéarate de magnésium.

3. Utilisation de stéarate de magnésium selon la revendication 1 ou 2, **caractérisée en ce que** le véhicule pharmaceutiquement inactif a un diamètre de particule moyen (diamètre aérodynamique médian en masse, DAMM) d'environ 10 à 500 µm et la substance active pharmaceutique finement répartie sous forme de particules a un diamètre de particule moyen (mesuré en tant que DAMM) d'au maximum environ 10 µm.

4. Utilisation selon l'une des revendications 1 à 3, **caractérisée en ce que** le véhicule pharmaceutiquement inactif, la substance active pharmaceutique finement répartie et le stéarate de magnésium sont présents sous la forme d'un mélange interactif.

5. Utilisation selon l'une des revendications 1 à 4, **caractérisée en ce que** la formulation de poudre sèche peut être obtenue en mélangeant du lactose monohydraté ayant un diamètre de particule moyen (DAMM) d'environ 10 à 500 µm utilisé comme véhicule, la substance active pharmaceutique finement répartie ayant un diamètre de particule moyen (mesuré en tant que DAMM) d'au maximum 10 µm, et le stéarate de magnésium sous forme de poudre de manière telle que les particules desdits constituants restent telles quelles, en dehors de l'adhérence aux particules de véhicule.

6. Utilisation selon l'une des revendications 1 à 5, **caractérisée en ce que** le stéarate de magnésium sous forme de poudre dans la formulation de poudre sèche est présent à une concentration de 0,4 à 0,8 % en poids, préférablement de 0,5 à 0,75 % en poids.

7. Utilisation selon l'une des revendications 1 à 6, **caractérisée en ce que** la substance active pharmaceutique est un bêta-mimétique et/ou un anticholinergique et/ou un corticostéroïde ou un sel ou un ester pharmaceutiquement acceptable.

8. Utilisation selon la revendication 7, dans laquelle le corticostéroïde est la fluticasone.

9. Utilisation selon l'une des revendications 1 à 8, **caractérisée en ce que** la formulation de poudre sèche comprend un corticostéroïde en combinaison avec un bêta-mimétique comme substance active pharmaceutique, préférablement un corticostéroïde sélectionné dans le groupe constitué du ciclésonide, du rofléponide, du propionate de fluticasone, du furoate de mométasone et de l'étabonate de lotéprednol en combinaison avec un bêta-mimétique sélectionné dans le groupe constitué du fumarate de formotérol, du tartrate de formotérol, du sulfate de lévalbutérol et du xinafoate de salmétérol.

10. Utilisation selon l'une des revendications 1 à 9, **caractérisée en ce que** la formulation de poudre sèche contient aussi une proportion de particules de véhicule inhalables en plus des particules de véhicule non inhalables, en particulier une proportion de 0,1 à 10 % en poids de lactose monohydraté micronisé en plus des particules de véhicule de lactose monohydraté non inhalables.
